# EUROPEAN PATENT APPLICATION

(11) **EP 2 863 229 A1**
(43) Date of publication of application: **22.04.2015**
(21) Application number: 13188679.8
(22) Date of filing: 15.10.2013
(51) Int. Cl.: G01N 33/84, A61B 5/055, A61K 49/06, G01N 24/08, G01R 33/56, A61K 49/10

(54) **pH-biosensors based on compounds with pH-sensitive enolic groups for magnetic resonance imaging and spectroscopy and their uses**

(71) Applicant: Technische Universität München, 80333 München (DE)
(72) Inventor: Schilling, Franz, 80805 Munich (DE); Glaser, Steffen, 85748 Garching (DE); Düwel, Stephan, 80779 Munich (DE); Gersch, Malte, 80803 Munich (DE)
(74) Representative: Engelhard, Markus

(57) **Abstract**

The present invention relates to the use of compounds with at least one pH-sensitive chemical shift for determining pH and/or measuring pH changes in magnetic resonance. More specifically, the present invention is related to compounds with at least one pH-sensitive chemical shift, such compound being selected from pyruvic acid and its metabolites, compounds produced from pyruvic acid after interaction with acid, and compounds comprising at least one enolic group whose pKₐ value is lowered through effects of at least one neighboring group into a physiological and/or pathological pH-range, and wherein the compound exhibits at least one pH-sensitive chemical shift in an NMR spectrum. The present invention further relates to biosensors comprising at least one of the compounds. The present invention is furthermore related to *in vitro* and *in vivo* methods for determining pH and/or measuring pH changes using the compounds or biosensors. The present invention also relates to methods of diagnosing and/or monitoring treatment of a disease causing changes in pH wherein the compounds or biosensors are applied. The present invention also relates to use of the compounds or biosensors in quality control of food or in the examination of plants and organisms.

## Description

The present invention relates to the use of compounds with at least one pH-sensitive chemical shift for determining pH and/or measuring pH changes in magnetic resonance. More specifically, the present invention is related to compounds with at least one pH-sensitive chemical shift, such compound being selected from pyruvic acid and its metabolites, compounds produced from pyruvic acid after interaction with acid, and compounds comprising at least one enolic group whose pKₐ value is lowered through effects of at least one neighboring group into a physiological and/or pathological pH-range, and wherein the compound exhibits at least one pH-sensitive chemical shift in an NMR spectrum. The present invention further relates to biosensors comprising at least one of the compounds. The present invention is furthermore related to *in vitro* and *in vivo* methods for determining pH and/or measuring pH changes using the compounds or biosensors. The present invention also relates to methods of diagnosing and/or monitoring treatment of a disease causing changes in pH wherein the compounds or biosensors are applied. The present invention also relates to use of the compounds or biosensors in quality control of food or in the examination of plants and organisms.

### BACKGROUND OF THE INVENTION

In mammalian tissues, intra- and extracellular pH are regulated in a dynamic steady state driven by metabolic acid production, export of H⁺ from cells, and diffusion of these H⁺ equivalents from the site of production to the blood, where they are buffered by an open and dynamic CO₂/HCO₃⁻ system. Although this balance is quite robust, it can be altered in many pathological states, notably cancers, renal failure, ischemia, inflammation and chronic obstructive pulmonary disease (Gillies et al., 2004).

In the field of magnetic resonance various pH -sensor molecules have been developed whose ¹H, ¹⁹F, or ³¹P resonance frequencies (chemical shifts) change with pH (Gillies et al., 2004; Arnold et al., 1984; De Leon et al., 2009; Morikawa et al., 1993 and Zhang et al., 2010). Those methods allow for a non-invasive detection of both intra- and extracellular pH. However, they suffer from low sensitivity and are thus not suitable for highly spatially resolved pH mapping by magnetic resonance imaging (MRI).

For this reason other classes of exogenous pH-sensitive contrast agents were developed based on pH-dependent magnetization transfer between water and a contrast agent (mostly lanthanoid complexes) or based on pH-dependent relaxation properties of gadolinium complexes (Gillies et al., 2004; De Leon et al., 2009; Aime et al., 2002; Castelli et al, 2013). The main disadvantages of these pre-clinically applied methods are that they require either long irradiation with radiofrequency waves or an exact determination of contrast agent concentration. Therefore, it is unclear, whether those techniques will translate into clinical applications. Long radiofrequency irradiation is mostly prohibited by specific absorption rate (SAR) limitations in the clinic and gadolinium-/lanthanoid-complexes are restricted in clinical use due to their toxicity.

In 2003 dissolution dynamic nuclear polarization (DNP) revolutionized magnetic resonance spectroscopy by bringing nuclear spins in a so-called hyperpolarized state leading to a sensitivity gain by more than four orders of magnitude. This allows to image formerly insensitive nuclei such as ¹³C (Ardenkjaer-Larsen et al., 2003). A technique for mapping pH spatially by taking the ratio of hyperpolarized bicarbonate (HCO₃⁻) to CO₂ also relies on DNP which represents the current state-of-the-art method in NMR-based pH measurements (Gallagher et al., 2008). Disadvantages of this method are the signal-to-noise-ratio-limited accuracy in the measurement of peak intensities and the influence of enzyme concentration (e.g. carbonic anhydrase) on the measurement of pH (Schroeder et al., 2010).

Hyperpolarized [1-¹³C]-pyruvate is described to be used for detecting tumor response to chemotherapy treatment in lymphoma-bearing mice (Day et al., 2007) and is currently being used in patients as a novel contrast agent in a clinical study at the University of San Francisco for applications in metabolic imaging of prostate carcinoma (Nelson et al., 2013 -1 and Nelson et al., 2013 -2). This first-in-man imaging study evaluated the safety and feasibility of hyperpolarized [1-¹³C]-pyruvate as an agent for noninvasively characterizing alterations in tumor metabolism for patients with prostate cancer. It was possible to evaluate the distribution of [1-¹³C]-pyruvate and its metabolic product lactate in a matter of seconds, as well as the flux of pyruvate to lactate.

WO 2008/020764 A1 discloses methods of ¹³C-MR imaging and/or ¹³C-MR spectroscopy of cell death using an imaging medium which comprises hyperpolarized ¹³C-pyruvate. WO 2008/020765 A2 discloses an imaging medium containing lactate and hyperpolarized ¹³C-pyruvate, a method to produce said imaging medium, use of said imaging medium and methods of ¹³C-MR imaging and/or ¹³C-MR spectroscopy wherein said imaging medium is used. WO 2011/138269 A1 discloses a hyperpolarized MR imaging medium comprising hyperpolarized [¹³C, ²H]lactate and a method of ¹³C-MR detection for the determination of lactate dehydrogenase (LDH) activity.

Besides magnetic resonance, optical methods such as fluorescence microscopy_ENREF_11 (Hassan et al., 2007) or radioactive tracers_ENREF_12 (Vavere et al., 2009) in positron-emission-tomography (PET) can potentially be used for pH-mapping.

Although many non-invasive pH-mapping methods exist, none of these made the translation from preclinical studies to the clinic.

There is a need in the art for improved means and methods for measuring pH and/or pH changes, preferably in real-time and/or in a spatial resolution, especially *in vivo.*

### SUMMARY OF THE INVENTION

According to the present invention this object is solved by the use of a compound with at least one pH-sensitive chemical shift for determining pH and/or measuring pH changes, wherein the compound is selected from pyruvic acid and its metabolites, compounds produced from pyruvic acid after interaction with acid, and compounds comprising at least one enolic group whose pKₐ value is lowered through effects of at least one neighboring group into a physiological and/or pathological pH-range.

According to the present invention this object is solved by a biosensor for determining pH and/or measuring pH changes, comprising
at least one compound with at least one pH-sensitive chemical shift of the present invention,
optionally, a reference compound,
optionally, pharmaceutically acceptable carriers and/or excipients.

According to the present invention this object is solved by providing the compound of the present invention or the biosensor of the present invention for use in *in vivo* magnetic resonance imaging (MRI) or magnetic resonance spectroscopy (MRS).

According to the present invention this object is solved by providing the compound of the present invention or the biosensor of the present invention for use in diagnosing and/or monitoring treatment of a disease causing changes in pH.

According to the present invention this object is solved by using the compound of the present invention or the biosensor of the present invention as pH sensor for use in *in-vitro* magnetic resonance imaging (MRI) or magnetic resonance spectroscopy (MRS)..

According to the present invention this object is solved by an *in-vitro* method for determining pH and/or measuring pH changes, preferably in real-time, comprising the steps of
(i) providing a sample,
(ii) adding a compound with at least one pH-sensitive chemical shift of the present invention or a biosensor of the present invention to the sample,
(iii) performing magnetic resonance imaging (MRI) or magnetic resonance spectroscopy (MRS) and thereby determining the pH or pH changes of or in the sample by obtaining a chemical shift difference between at least one pH-sensitive chemical shift of the compound and a pH-independent chemical shift, such pH-independent chemical shift acting as a reference chemical shift, or by measurement of the absolute chemical shift, or by measuring chemical shift differences involving at least one pH-sensitive chemical shift, preferably over time.

According to the present invention this object is also solved by an *in-vivo* method for determining pH and/or measuring pH changes, preferably in real-time, comprising the steps of
(i) applying or administering a compound with at least one pH-sensitive chemical shift of the present invention or a biosensor of the present invention to the body of a patient or non-human animal,
(ii) performing magnetic resonance imaging (MRI) or magnetic resonance spectroscopy (MRS) and thereby determining one or several pH values or pH changes of or in the body of said patient or non-human animal by obtaining achemical shift difference between at least one pH-sensitive chemical shift of the compound and a pH-independent chemical shift, such pH independent chemical shift acting as a reference chemical shift or by measurement of the absolute chemical shift, or by measuring chemical shift differences involving at least one pH-sensitive chemical shift, preferably over time.

According to the present invention this object is solved by a method of diagnosing and/or monitoring treatment of a disease causing changes in pH, comprising the steps of
(i) applying or administering a compound with at least one pH-sensitive chemical shift of the present invention or a biosensor of the present invention to the body of a patient or non-human animal,
(ii) performing magnetic resonance imaging (MRI) or magnetic resonance spectroscopy (MRS) and thereby determining one or several pH values or pH changes of or in the body of said patient or non-human animal by obtaining the chemical shift difference between at least one pH-sensitive chemical shift of the compound and a pH-independent chemical shift, such pH-independent chemical shift acting as a reference chemical shift, or by measurement of the absolute chemical shift, or by measuring chemical shift differences involving at least one pH-sensitive chemical shift over time,
(iii) calculating pH maps based on spatially resolved pH values or pH changes determined in step (ii).

According to the present invention this object is solved by using the compound of the present invention or the biosensor of the present invention in quality control of food or in the examination of plants and organisms.

### DESCRIPTION OF THE PREFERRED EMBODIMENTS OF THE INVENTION

Before the present invention is described in more detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art. For the purpose of the present invention, all references cited herein are incorporated by reference in their entireties.

*pH biosensors based on pyruvic acid and its metabolites, compounds produced from pyruvic acid after interaction with acid, or on compounds with pH-sensitive enolic group(s)* As discussed above, the present invention provides the use of a compound with at least one pH-sensitive chemical shift for determining pH and/or measuring pH changes.

The inventors have surprisingly found that it is possible to make use of the pH-dependent displacement of chemical shifts in compounds for determining one or several pH values and/or for measuring pH changes. In particular, this concerns the displacement of pH sensitive ¹³C chemical shifts in ¹³C-magnetic resonance imaging and/or ¹³C magnetic resonance spectroscopy.

The present invention thus provides for the use of a compound which shows a pH-dependent displacement of at least one pH-sensitive chemical shift for determining pH and/or measuring pH changes. The term "displacement of a chemical shift, ", as used herein is meant to refer to a change in position of the respective chemical shift. In this context, "displacement of a chemical shift" is preferably meant to refer to a change in position of a ¹³C chemical shift.

Preferably, a compound with at least one pH-sensitive chemical shift comprises one or more pH-sensitive chemical shifts, such as two, three, four or more.

Here, a novel pH biosensor is presented (that is based on a compound with at least one pH-sensitive chemical shift, such as zymonic acid, its analogs or further compounds produced by acid treatment/interaction from pyruvic acid) for magnetic resonance that is very sensitive to pH-changes in a physiologically and/or pathologically relevant pH range. This novel sensor acts independently of its concentration and enzymatic reactions and therefore allows a very accurate pH mapping at high spatial resolution making it a promising probe for the translation to the clinic.

As used herein "magnetic resonance" refers to the observation of Larmor precession in a magnetic field (see Ernst, 1997 and de Graaf, 2007), and includes measurements at a NMR spectrometer, an NMR microimaging system, an MRI scanner, a low-field NMR device, microfluidic arrays ("NMR on a chip"), and/or combinations thereof. Measurement includes all variations of spatially and/or spectrally resolved magnetic resonance techniques, such as magnetic resonance spectroscopy (MRS), magnetic resonance imaging (MRI), or magnetic resonance spectroscopic imaging (MRSI).

According to the present invention, said compound with at least one pH-sensitive chemical shift is selected from pyruvic acid and its metabolites, compounds produced from pyruvic acid after interaction with acid and compounds comprising at least one enolic group whose pKₐ value is lowered through effects of at least one neighboring group into a physiological and/or pathological pH range.

As used herein "chemical shift" in magnetic resonance refers to the resonance of a nucleus relative to a suitable standard, such as tetramethylsilane (TMS).

Preferably, the compounds comprise at least one enolic group (preferably one or more enolic groups, such as two, three, four or more) whose pKa-value is lowered through effects of one or more neighboring groups into a physiological and/or pathological pH-range, such as two, three, four or more neighboring groups.

A "neighboring group to a specific group" refers to a functional group (such as a carboxylic group or ester group in Zymonic acid) connected up to seven bonds away from such specific group, e.g. from the enolic group.

A "physiological and/or pathological pH-range" or "physiologically and/or pathologically relevant pH-range" refers to pH ranges of from about 5 to about 9. Preferably, a "physiological pH-range" is from about 6 to about 8.

According to the present invention, said compound with at least one pH-sensitive chemical shift exhibits at least one NMR resonance with a pH-sensitive chemical shift in an NMR spectrum.

A preferred example of said compound with at least one pH-sensitive chemical shift is zymonic acid.

Zymonic acid is also referred to as 2,5-dihydro-4-hydroxy-2-methyl-5-oxo-2-furancarboxylic acid.
IUPAC name: 4-hydroxy-2-methyl-5-oxo-2,5-dihydrofuran-2-carboxylic acid
Traditional IUPAC name: 4-hydroxy-2-methyl-5-oxofuran-2-carboxylic acid
(see http://www.hmdb.ca/metabolites/HMDB31210))

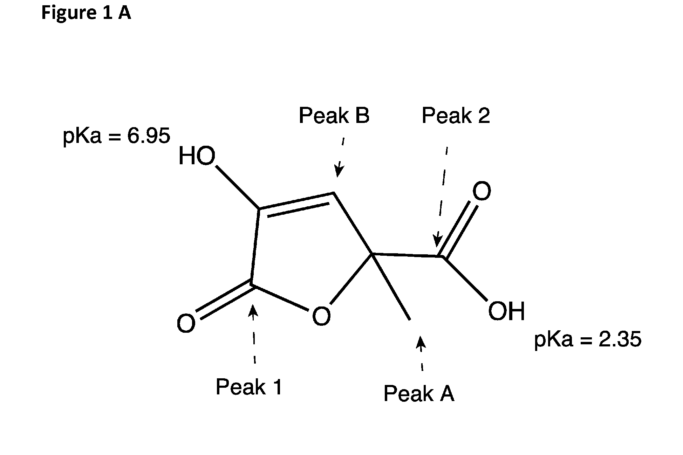

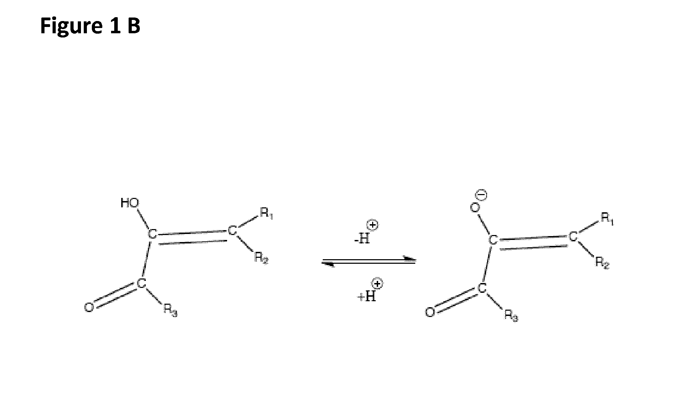

The inventors have found that zymonic acid exhibits a pH-dependent chemical shift for some of its ¹³C- and ¹H-resonances (marked in Figure 1). In Figure 1, zymonic acid's pKₐ-values are shown and assigned to the relevant proton donating groups. The pKₐ of the enolic group is 6.95.

Zymonic acid has been mentioned in the 1950s for the first time and can be produced by yeast bacteria from glucose or can originate from a ring closure of parapyruvate molecules which in turn can originate from pyruvic acid_ENREF_13 (Bloomer et al., 1970 -1; Bloomer et al., 1970 -2; Stodola et al., 1952; de Jong, 1901). Zymonic acid is used as a flavor constituent for confectionary and in the tobacco industry and is therefore not toxic when administered *in vivo.*

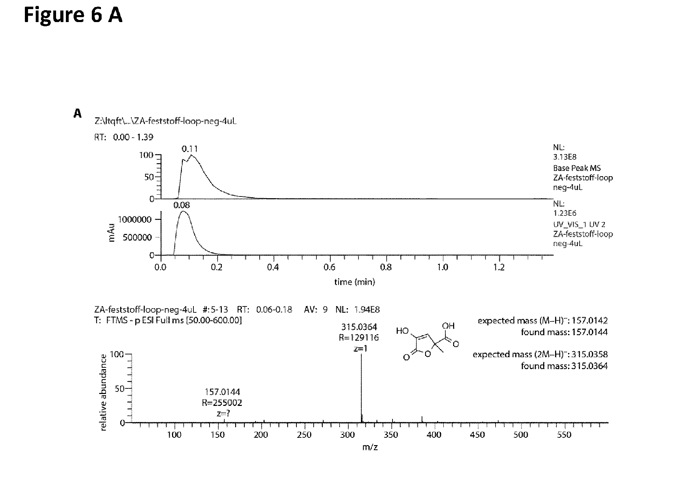

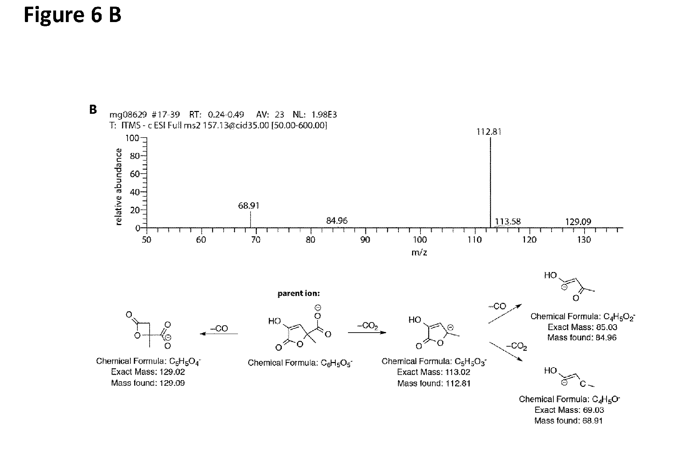

Zymonic acid is an extremely sensitive pH biosensor for magnetic resonance spectroscopy (MRS) and magnetic resonance imaging (MRI) and exhibits the following properties, which sets zymonic acid apart from other non-invasive methods to measure pH:
(a) Zymonic acid exhibits the highest pH-dependent change in chemical shift measured to date with ¹³C shifts up to 2.35 ppm/pH in the physiologically and/or pathologically relevant range from pH 5 to pH 9 and is, thus, suitable for a very accurate noninvasive pH determination using magnetic resonance spectroscopy/imaging. It should be noted that such pH-dependent change in chemical shift typically is a change in the position of the respective resonance peak of this chemical shift in an NMR spectrum.
(b) Unlike some other pH measurement methods, the pH determination is robustly performed using a relative or absolute frequency encoding. In contrast, the amplitude encoding used in the bicarbonate method (i.e. a change in intensity) (Gallagher et al., 2008; Schroeder et al., 2010) is prone to spatial or temporal fluctuations in concentration.
(c) ¹³C-labeled zymonic acid (see peaks 1 and 2 in Figure 6) can be produced from [1-¹³C]-pyruvate in a one-step synthesis. The two labeled carbons are exposed to a weak dipolar interaction and thus exhibit a long longitudinal relaxation time T₁. Hyperpolarization increases the polarization of the molecule by four to five orders of magnitude which enables pH imaging in the human body at low contrast agent concentration in the micromolar to millimolar concentration range and at the same time high spatial resolution with centimeter to sub-millimeter voxel size.

According to the present invention, the compound is preferably selected from
zymonic acid,
analogs of zymonic acid,
pyruvic acid and its metabolites,
compounds that are produced from pyruvic acid after interaction with acid,
diethyl oxaloacetic acid,
and their hydrates, salts, solutions, stereoisomers.

In one embodiment an "analog of zymonic acid" according to the invention is
- an ester, e.g. methyl ester,
- an ether,
- an amide,
- a fluorinated analog, e.g. a trifluoromethyl analog, or a
- deuterated analog.

In one embodiment, said analog is selected from

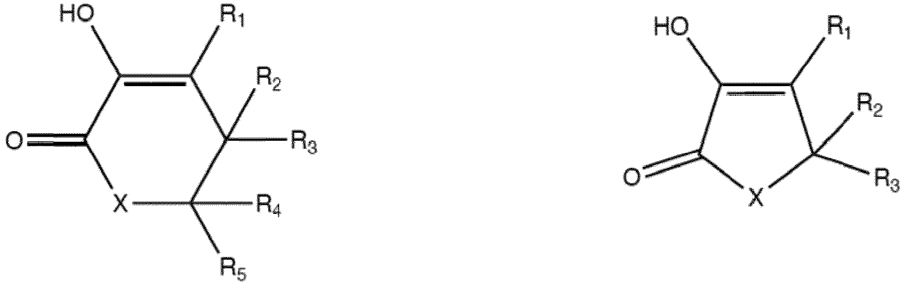

Wherein X is selected from CR₆R₇, O, NR₆, S, and wherein R₁ to R₇ is, at each occurrence, independently selected from H, alkyl, halogen, CN, methoxy, carboxy, aryl, e.g. benzyl, wherein, preferably, one of R₂ and R₃ is carboxy.

A "compound that is produced from pyruvic acid after interaction with acid" refers to any (other) compound that is produced when HCl or an other strong or weak acid (aqueous or as a gas) acts on pyruvic acid for some time, and which compound exhibits a pH sensitive chemical shift as defined herein. De Jong, 1901 and Montgomery & Webb, 1954 disclose a respective method of obtaining such compounds.

Another example for a compound with a pH sensitive chemical shift according to the present invention is diethyl oxaloacetic acid which has an enolic group with a pKa of 7.6 (Montgomery & Webb, 1954).

According to the present invention, bicarbonate as a metabolite of pyruvic acid is not encompassed by the invention.

### - pH sensitivity

A (acid) compound with at least one pH-sensitive chemical shift of the invention includes, inter alia, zymonic acid, its analogs and further compounds, as defined herein.

Preferably, the compound is ¹³C-labeled.
More preferably, the compound exhibits at least one pH-sensitive ¹³C-chemical shift, such as in the range of 170-180 ppm.

Preferably, the compound exhibits at least one pH-sensitive chemical shift sensitive in the physiological and/or pathological pH range, from about pH 5 to about pH 9.

In a preferred embodiment, the compound is hyperpolarized.

Hyperpolarization of NMR active ¹³C-nuclei may be achieved by different methods, which are for instance described in WO 98/30918, WO 99/24080 and WO 99/35508, and hyperpolarization methods are polarization transfer from a noble gas, "brute force", spin refrigeration, the parahydrogen method (parahydrogen induced polarisation (PHIP)) and dynamic nuclear polarization (DNP).

Preferably, the hyperpolarization is by dynamic nuclear polarization (DNP).

The term "hyperpolarized" refers to a nuclear polarization level in excess of 0.1%, more preferred in excess of 1% and most preferred in excess of 10%. The level of polarization may for instance be determined by solid state ¹³C-NMR measurements, such as in solid hyperpolarized ¹³C-pyruvic acid or ¹³C-zymonic acid (or other compounds), e.g. obtained by dynamic nuclear polarization (DNP) of ¹³C-pyruvic acid or ¹³C-zymonic acid. The solid state ¹³C-NMR measurement preferably consists of a simple pulse-acquire NMR sequence using a low flip angle. The signal intensity of the hyperpolarized ¹³C-pyruvic acid/zymonic acid in the NMR spectrum is compared with signal intensity in a NMR spectrum acquired before the polarization process. The level of polarization is then calculated from the ratio of the signal intensities before and after polarization. In a similar way, the level of polarization for dissolved hyperpolarized ¹³C-pyruvic acid or ¹³C-zymonic acid (or other compounds) may be determined by liquid state NMR measurements. Again the signal intensity of the dissolved hyperpolarized ¹³C-pyruvic acid or ¹³C-zymonic acid is compared with the signal intensity before polarization. The level of polarization is then calculated from the ratio of the signal intensities before and after polarization.

Preferably, the (acid) compound with one or more pH-sensitive chemical shifts of the invention has a pKₐ value in a physiological and/or pathological pH range (from about pH 5 to about pH 9).

Preferably, the carbon(s) belonging to the pH-sensitive chemical shift(s) of the (acid) compound with one or more pH-sensitive chemical shifts exhibit(s) a long longitudinal relaxation time T₁.

### - Chemical shift reference

Preferably, a reference chemical shift which is pH-insensitive, i.e. not pH-sensitive and, thus, exhibits no change in chemical shift upon change of pH, is required. This is typically provided in the form of a further compound, the "reference compound", that is added to or also present in a sample.

Alternatively, a chemical shift with a different chemical-shift-pH-correlation can serve as a reference. This may, e.g., be a chemical shift within the compound according to the present invention.

The reference chemical shift can be an endogenous reference or an exogenous reference or a chemical shift of the compound itself or its metabolites.

In one embodiment, the (acid) compound with one or more pH-sensitive chemical shifts of the invention furthermore exhibits at least one chemical shift that is not pH-sensitive, preferably at least one pH-insensitive ¹³C-chemical shift. (endogenous reference)

In one embodiment, the (acid) compound with one or more pH-sensitive chemical shifts of the invention furthermore exhibits at least one chemical shift that is pH-sensitive with a different chemical-shift-pH-correlation, preferably at least one pH-sensitive ¹³C-peak. (endogenous pH-sensitive reference)

In one embodiment, a reference compound is used. The reference compound is a compound which does not exhibit pH-sensitive shift(s) (exogenous reference).

Preferably the reference compound is ¹³C-labeled and preferably exhibits at least one pH-insensitive ¹³C-peak.

A preferred reference compound is ¹³C urea (or ¹³C-pyruvate, or ¹³C-pyruvate hydrate, or ¹³C-parapyruvate, or ¹³C-lactate, or ¹³C-alanine ).

For example, the reference compound is obtained in that a substance or compound (such as urea) is co-polarized at the same time when the compound with one or more pH-sensitive chemical shift of the invention is hyperpolarized.

As discussed above, the present invention provides an *imaging medium,*
comprising
at least one compound with at least one pH-sensitive chemical shift as defined herein,
optionally, pharmaceutically acceptable carriers and/or excipients, such as an aqueous carrier, like a buffer.

Preferably, the imaging medium is a magnetic resonance (MR) imaging medium.

The term "imaging medium" refers to a liquid composition comprising at least one compound with one or more pH-sensitive chemical shifts of the present invention (such as hyperpolarized ¹³C-zymonic acid or hyperpolarized ¹³C-pyruvate) as the MR active agent. The imaging medium according to the invention may be used as imaging medium in MR imaging or as MR spectroscopy agent in MR spectroscopy. The imaging medium according to the invention may be used as imaging medium for *in vivo* MR imaging and/or spectroscopy, i.e. MR imaging and/or spectroscopy carried out on living human or non-human animal beings. Further, the imaging medium according to the invention may be used as imaging medium for *in vitro* MR imaging and/or spectroscopy, e.g. for determining pH and/or pH changes in cell cultures or *ex vivo* tissues. Cell cultures may be derived from cells obtained from samples derived from the human or non-human animal body, like for instance blood, urine or saliva, while *ex vivo* tissue may be obtained from biopsies or surgical procedures.

In one embodiment, the imaging medium preferably comprises in addition to the MR active agent an aqueous carrier, preferably a physiologically tolerable and pharmaceutically accepted aqueous carrier, like water, a buffer solution or saline. Such an imaging medium may further comprise conventional pharmaceutical or veterinary carriers or excipients, e.g. formulation aids such as are conventional for diagnostic compositions in human or veterinary medicine.

In one embodiment, the imaging medium preferably comprises in addition to the MR active agent a solvent which is compatible with and used for *in vitro* cell or tissue assays, for instance DMSO or methanol or solvent mixtures comprising an aqueous carrier and a nonaqueous solvent, for instance mixtures of DMSO and water or a buffer solution or methanol and water or a buffer solution.

Preferably, at least one compound with at least one or more pH-sensitive chemical shifts is used in concentrations of up to 1 M, preferably 0.1 to 100 mM, such as 10 to 50 mM, in the imaging medium.

As discussed above, the present invention provides a *biosensor* for determining pH and/or measuring pH changes,
comprising
at least one compound with at least one pH-sensitive chemical shift as defined herein,
optionally, a reference compound,
optionally, pharmaceutically acceptable carriers and/or excipients.

In one embodiment, the reference compound is a compound which does not exhibit pH-sensitive chemical shift(s) (exogenous reference chemical shift, as described above).

Preferably, the reference compound is ¹³C-labeled and preferably exhibits at least one pH-insensitive ¹³C-chemical shift.

Alternatively, a chemical shift with a different chemical-shift-pH-correlation can serve as a reference (see definition of chemical shift reference above).

A preferred reference compound is ¹³C urea.

For example, the reference compound is obtained in that a substance or compound (such as urea) is co-polarized at the same time when the compound with one or more pH-sensitive chemical shifts of the invention is hyperpolarized.

Preferably, the at least one compound with at least one/one or more pH-sensitive chemical shift is used in concentrations of up to 1 M, preferably 0.1 to 100 mM, such as 10 to 50 mM, in the biosensor.

### Imaging and medical uses

As discussed above, the present invention provides the compound with at least one pH-sensitive chemical shift of the present invention (the imaging medium of the present invention) or the biosensor of the present invention for use in *in vivo* magnetic resonance imaging (MRI) or magnetic resonance spectroscopy (MRS).

As discussed above, the present invention provides the compound with at least one pH-sensitive chemical shift of the present invention (the imaging medium of the present invention) or the biosensor of the present invention for use in diagnosing and/or monitoring treatment of a disease causing changes in pH.

Thereby, the progress of a disease and/or the treatment of a disease can be monitored.

Preferably, a "disease causing changes in pH" is selected from cancers, inflammation, ischemia, renal failure and chronic obstructive pulmonary disease.

Preferably, the imaging is real-time.

Preferably, the uses comprise the resolution of the spatial pH distribution, preferably, comprising the use of frequency encoding techniques, such as all methods of chemical shift imaging (CSI) and phase sensitive encodings of chemical shifts (as e.g. used in non-invasive spatially resolved temperature measurements using changes in proton resonance frequencies (0.01 ppm/°C)). See Rieke et al., 2004.

As discussed above, the present invention provides the use of the compound of the present invention as pH sensor for *in vitro* NMR-spectroscopy.

Preferably, the use comprises response-to-treatment monitoring of treatments applied to cell lines.

### Methods for determining pH and/or measuring pH changes

As discussed above, the present invention provides an *in-vitro* as well as an *in-vivo* method for determining pH and/or measuring pH changes.

Said *in-vitro* method of the present invention comprises the steps of
(i) providing a sample,
(ii) adding a compound with at least one pH-sensitive chemical shift of the present invention, an imaging medium of the present invention or a biosensor of the present invention to the sample,
(iii) performing magnetic resonance imaging (MRI) or magnetic resonance spectroscopy (MRS) and thereby determining the pH or pH changes of or in the sample by obtaining a chemical shift difference between at least one pH-sensitive chemical shift of the compound and a pH-independent chemical shift, such pH-independent chemical shift acting as a reference chemical shift, or by measurement of the absolute chemical shift, or by measuring chemical shift differences involving at least one pH-sensitive chemical shift.

Preferably, the sample is a cell culture sample, such as derived from a human or non-human body, *ex vivo* tissue, cell culture.

Preferably, step (iii) is carried out in an MRI scanner machine with MRS or MRSI capabilities or in a NMR spectrometer (such as with a microimaging head).

Preferably, the pH-independent chemical shift (acting as a reference chemical shift) is from the same compound, i.e. the compound with at least one pH-sensitive chemical shift (endogenous reference chemical shift, as described above), or from another substance (exogenous reference chemical shift, as described above), and is used as a pH-independent reference.

Alternatively, a chemical shift with a different chemical-shift-pH-correlation can serve as a reference.

Said *in-vivo* method of the present invention comprises the steps of
(i) applying or administering a compound with at least one pH-sensitive chemical shift of the present invention, an imaging medium of the present invention or a biosensor of the present invention to the body of a patient or non-human animal,
(ii) performing magnetic resonance imaging (MRI) and thereby determining one or several pH values or pH changes of or in the body of said patient or non-human animal by obtaining a chemical shift difference between at least one pH-sensitive chemical shift of the compound and a pH-independent chemical shift, such pH-independent chemical shift acting as a reference chemical shift, or by measurement of the absolute chemical shift, or by measuring chemical shift differences involving at least one pH-sensitive chemical shift.

Preferably, the *in-vivo* method is a real-time method.

In one embodiment, the patient is a human.

Preferably, the patient can be diagnosed with a disease causing changes in pH or the treatment of a disease causing changes in pH can be monitored.

Preferably, "a disease causing changes in pH" is selected from cancers, inflammation, ischemia, renal failure and chronic obstructive pulmonary disease.

Preferably, the pH-independent chemical shift (reference chemical shift) is from the same compound, i.e. the compound with at least one pH-sensitive chemical shift (endogenous reference chemical shift, as described above), or from another substance (exogenous reference chemical shift, as described above), and is used as a pH-independent reference.

Alternatively, a chemical shift with a different chemical-shift-pH-correlation can serve as a reference.

Preferably, the *in-vitro* and/or the *in-vivo* method comprises the resolution of the spatial pH distribution and, thus, obtaining spatially resolved NMR spectra,
preferably, comprising the use of frequency encoding techniques, such as all methods of chemical shift imaging (CSI) and phase sensitive encodings of chemical shifts (as e.g. used in non-invasive spatially resolved temperature measurements using changes in proton resonance frequencies (0.01 ppm/°C)). See Rieke et al., 2004.

### Methods for diagnosing and/or monitoring treatment

As discussed above, the present invention provides a method of diagnosing and/or monitoring treatment of a disease causing changes in pH.

Said method comprises the steps of
(i) applying or administering a compound with at least one pH-sensitive chemical shift of the present invention (an imaging medium of the present invention) or a biosensor of the present invention to the body of a patient or non-human animal,
(ii) performing magnetic resonance imaging (MRI) or magnetic resonance spectroscopy (MRS) and thereby determining one or several pH values or pH changes of or in the body of said patient or non-human animal by obtaining the chemical shift difference between at least one pH sensitive chemical shift of the compound and a pH-independent chemical shift, such pH-independent chemical shift acting as a reference chemical shift, or by measurement of the absolute chemical shift, or by measuring chemical shift differences involving at least one pH-sensitive chemical shift over time,
(iii) calculating pH maps based on spatially resolved pH values or pH changes determined in step (ii).

Preferably, step (iii) comprises
comparing said relative chemical shifts to predetermined calibration curves of the compound with at least one pH-sensitive chemical shift in solutions with known pH.

In one embodiment, the method further comprises
hyperpolarizing the compound with at least one pH-sensitive chemical shift before application or administration to the body of the patient.

Thereby, the compound is hyperpolarized by any hyperpolarization methods, such as dissolution dynamic nuclear polarization (DNP) or parahydrogen induced polarisation (PHIP).

Preferably, "a disease causing changes in pH" is selected from cancers, inflammation, ischemia, renal failure and chronic obstructive pulmonary disease.

Thereby, the progress of a disease and/or the treatment of a disease can be monitored.

Preferably, the method comprises magnetic resonance tomography (MRT).

Preferably, the imaging is real-time.

Preferably, the method comprises the resolution of the spatial pH distribution and, thus, obtaining spatially resolved NMR spectra,
preferably, comprising the use of frequency encoding techniques, such as all methods of chemical shift imaging (CSI) and phase sensitive encodings of chemical shifts (as e.g. used in non-invasive spatially resolved temperature measurements using changes in proton resonance frequencies (0.01 ppm/°C)). See Rieke et al., 2004.

### Further uses

As discussed above, the present invention provides the use of a compound of the present invention or a biosensor of the present invention as in quality control of food or in the examination of plants and organisms.

### Further description of a preferred embodiment

Local changes of pH in the human body are triggered by many pathologies that overrule natural pH regulatory mechanisms, in particular tumors, inflammation, and ischemia, but also renal failure and chronic obstructive pulmonary disease. The spatially resolved, robust, and non-invasive method for the exact measurement of local pH and its means, described herein, therefore offer improved means for preclinical and clinical applications both for diagnostics and therapeutical purposes, such as monitoring response-to-treatment. Furthermore, applications range from quality control of food to the examination of plants and organisms.

Here, a novel pH biosensor is presented (that is based on compounds with at least one pH-sensitive chemical shift, such as zymonic acid or its analogs) for magnetic resonance that is very sensitive to pH-changes in a physiologically and/or pathologically relevant pH range. This novel sensor acts independently of its concentration and enzymatic reactions and therefore allows a very accurate pH mapping at high spatial resolution making it a promising probe for the translation to the clinic.

The invention is based on the fact that the chemical compound zymonic acid exhibits a pH-dependent chemical shift for some of its ¹³C- and ¹H-resonances (marked in Figure 1). Zymonic acid has been discovered and named in the 1950s for the first time and can be produced by yeast bacteria from glucose or can originate from a ring closure of parapyruvate molecules which in turn can originate from pyruvic acid_ENREF_13 (Bloomer et al., 1970 -1; Bloomer et al., 1970 -2; Stodola et al., 1952). Zymonic acid is used as a flavor constituent for confectionary and in the tobacco industry and is therefore most likely not toxic when administered *in vivo.*

Zymonic acid is an extremely sensitive pH biosensor for magnetic resonance spectroscopy and magnetic resonance tomography and exhibits the following properties, which sets zymonic acid apart from other non-invasive methods to measure pH:
(a) Zymonic acid exhibits the highest pH-dependent change in chemical shift measured to date with ¹³C shifts up to 2.35 ppm/pH in the physiologically and/or pathologically relevant range from pH 5 to pH 9 and is thus suitable for a very accurate noninvasive pH determination using magnetic resonance tomography/spectroscopy.
(b) Unlike some other pH measurement methods, the pH determination is robustly performed using a relative or absolute frequency encoding as long as a pH independent reference, e.g. urea or a pH independent chemical shift of zymonic acid itself, is present in the sample. In contrast, the amplitude encoding used in the bicarbonate method is prone to spatial or temporal fluctuations in concentration.
(c) ¹³C-labeled zymonic acid (see chemical shifts 1 and 2 in Figure 6) can be produced from [1-¹³C]-pyruvate in a one-step synthesis. The two carboxyl groups are exposed to a weak dipolar interaction and thus exhibit a long longitudinal relaxation time T₁. Hyperpolarization increases the polarization of the molecule by a factor 50,000 which enables pH imaging in the human body at low contrast agent concentration and at the same time high spatial resolution.

Medical applications for pH imaging with this new pH sensor are extremely numerous since many pathologies cause changes in pH. Good examples are tumors, inflammation and ischemia, but also renal failure and chronic obstructive pulmonary disease. Furthermore, the application as a very precise pH sensor for *in vitro* NMR-spectroscopy is interesting, e.g. for response-to-treatment monitoring of treatments applied to cell lines.

The pH-dependent change in chemical shifts of zymonic acid can be used in magnetic resonance tomography to resolve the spatial pH distribution for which established frequency encoding techniques can be used. This includes all spectrally resolving variations of chemical shift imaging (CSI) as well as phase sensitive encodings of chemical shifts as e.g. used in non-invasive spatially resolved temperature measurements using changes in proton resonance frequencies (0.01 ppm/°C).

The following examples and drawings illustrate the present invention without, however, limiting the same thereto.

### BRIEF DESCRIPTION OF THE DRAWINGS

**Figure 1****.**
   (A) *Schematic depiction of zymonic acid.*
      Shown are zymonic acid's pKa-values assigned to the relevant proton donating groups. Two exemplary ¹³C resonances, which show a pH-dependent change (or displacement) in chemical shift, are marked in light grey and numbered 1 and 2 according to their increasing ¹³C-NMR resonance frequency (the pH-dependent proton resonances are marked in darker grey and numbered A and B according to their increasing ¹H-NMR resonance frequency). It should be noted that the two ¹³C resonances shown here are examples of pH-sensitive chemical shifts; however, also the other carbon atoms in zymonic acid, if ¹³C-labelled, will show such pH-sensitive chemical shifts.
   (B) Schematic depiction of pKa value
      Figure 1B) shows the protonated and unprotonated forms of compounds such as zymonic acid. Shown on the left is the protonated form, and on the right the deprotonated form.
**Figure 2****.**
   (**A**) pH dependent ¹³C chemical shifts from peaks 1 and 2 in ¹³C-NMR spectra of zymonic acid.
   (**B**) pH dependent ¹³C chemical shifts from peaks 1 and 2 in ¹³C-NMR spectra of zymonic acid and best fit straight lines. Peak 1 of zymonic acid shows a pH dependent change in chemical shift of approx. 2.1 ppm/pH, and peak 2 of approx. 1.1ppm/pH. The error bars are calculated from the pH values of the sample determined with a standard pH electrode before and after the NMR-measurement. The spectrum was referenced to ¹³C-urea at 0 ppm.

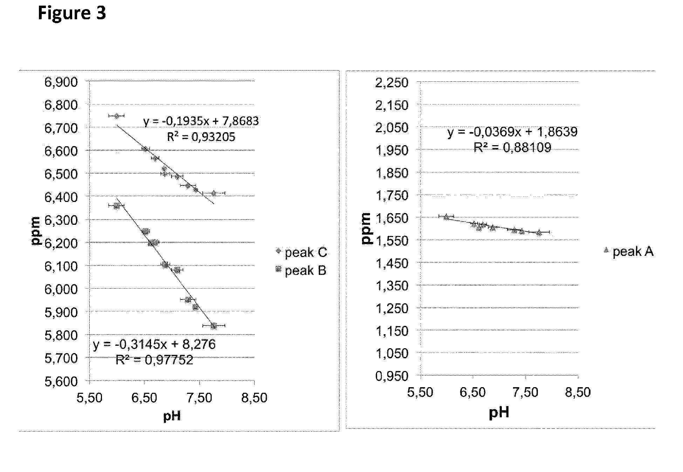

**Figure 3****.** *Proton chemical shifts of the proton peaks 4 and 5 of zymonic acid as a function of pH value.*
   (**A**) and (**B**): The additional peak C is produced during the synthesis of zymonic acid from pyruvate and can be assigned to a different, additional substance in the sample. The pH dependent change in chemical shift of peak B of zymonic acid is approx. -0.3 ppm/pH and peak A approx. -0.04 ppm/pH. Again, the error bars are calculated from the pH values of the sample determined with a standard pH electrode before and after the NMR-measurement. The spectrum was referenced to the proton peak of ¹³C-urea at 0 ppm.
**Figure 4****.**
   Injection of hyperpolarized [1-¹³C]-pyruvate in 40x10⁶ MCF-7 tumor cells in two series of measurements (**A**, **B**), in which the tumor cells were killed by addition of Triton-X100, which led to a gradually increasing acidification of the medium. The pH value was determined with a standard pH electrode after the NMR-measurement. The ¹³C spectra were referenced to the [1-¹³C]-pyruvate peak at 6.5 ppm. (C) Two peaks, which can be assigned to the hyperpolarized zymonic acid, show a strong pH dependent linear change in chemical shift with up to 2.35 ppm/pH.

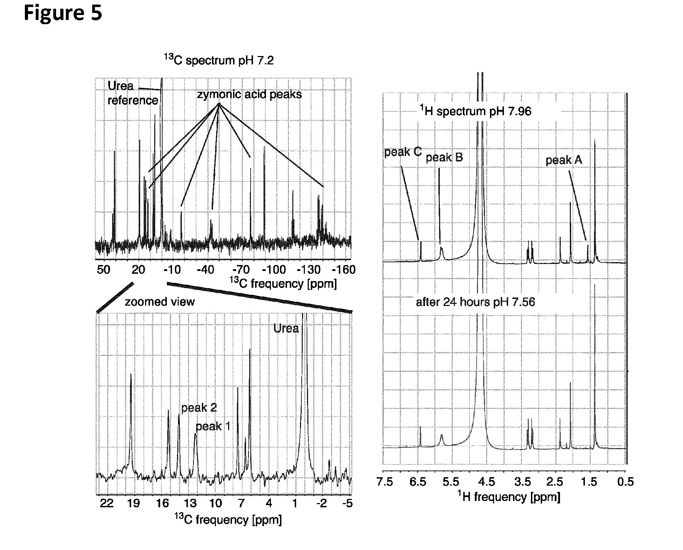

**Figure 5****.** *^{l3}C spectrum and ¹H spectrum of zymonic acid produced from [1-¹³C]-pyruvic acid.*
   An exemplary ¹³C spectrum (left column) and ¹H spectrum (right column) of zymonic acid produced from [1-¹³C]-pyruvic acid. The relevant peaks 1 to 5, which shift in dependency of the pH value of the sample, are marked. The proton spectra show a buffered solution of zymonic acid in water and D₂O, once measured right after preparation of the solution (top right) and 24h later (bottom right).
**Figure 6****.** *Confirmation of the structure of zymonic acid using mass spectrometry.*
   (**A**) The HR-MS-spectrum of the synthesized substance recorded with a Thermo Finnigan LTQ-FT confirms the total mass of the compound.
   (**B**) The MS/MS-spectrum of the synthesized substance recorded after CID-fragmentation on a Thermo Finigan LCQ-Fleet and the assignment of the observed fragments confirms the assumed structure.
Within the accuracy of the ion trap (±0.3 m/z), all peaks can be explained by elimination of carbon monoxide and carbon dioxide.

### EXAMPLES

### Materials and Methods

All experiments with zymonic acid described herein were conducted in the Department of Chemistry at Technische Universität München on a Bruker® 14.1 T NMR spectrometer with an AVANCE III console.

pH values were measured with a standard pH electrode.

### Production and characterization of zymonic acid

Zymonic acid was produced from pyruvic acid as described in the literature_ENREF_18 (De Jong, 1901). To this end, concentrated hydrochloric acid was added to pyruvic acid in a 1:1 volume ratio. The reaction mixture was then allowed to stand over concentrated sulfuric acid in an desiccator for two weeks at room temperature. Volatile compounds were removed *in vacuo* whereupon the yellow oil obtained showed crystallization. The yellow and strongly hygroscopic solid was then used without further purification.

The formula of zymonic acid is shown in Figure 1 (showing its pKa-values (Montgomery et al., 1954) assigned to the relevant proton donating groups and the pH-dependent ¹³C resonances and pH-dependent proton resonances).

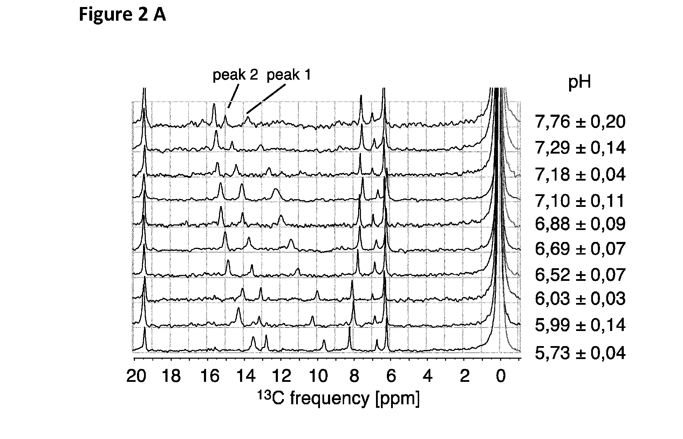

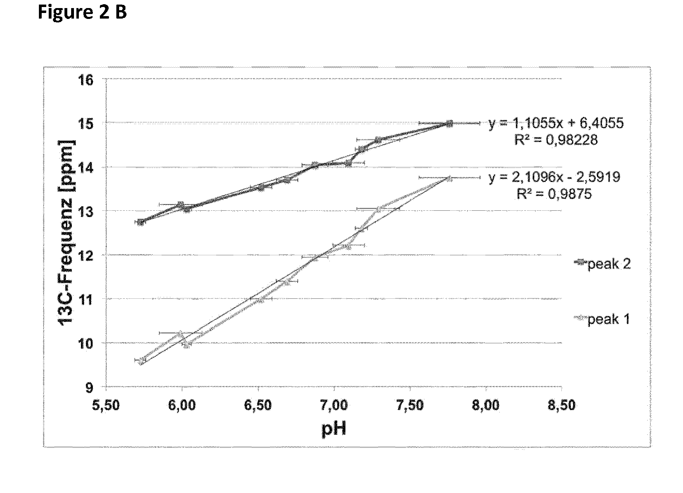

For the measurement of pH, calibration curves in 200 - 500 mM aqueous solutions of the reaction product were used in sodium phosphate buffer (1M). The pH was then adjusted by cautious addition of sodium hydroxide solution (10 M) or concentrated hydrochloric acid. 5-15% (v/v) D₂O and ¹³C-urea were added for referencing. The pH-dependent chemical shift of the relevant NMR peaks of zymonic acid is depicted in Figure 2. We did not observe hysteresis effects in the pH-dependent shift. In particular, Figure 2 shows the pH dependent ¹³C chemical shifts from peaks 1, 2 and 3 in ¹³C-NMR spectra of zymonic acid and their best fit straight lines. Peak 1 of zymonic acid shows a pH dependent change in chemical shift of approx. 2.1 ppm/pH, peak 2 of approx. 1.1ppm/pH and peak 3 of approx. 1.0 ppm/pH.

The assignment of the NMR-peaks of zymonic acid was done using NMR prediction software (ChemDraw®) and standard 1D- and 2D-NMR-spectroscopy (see Figure 5). In particular, Figure 5 shows an exemplary ¹³C spectrum (left column of Figure 5) and ¹H spectrum (right column of Figure 5) of zymonic acid produced from [1-¹³C]-pyruvic acid. The relevant peaks 1 to 5, which shift in dependency of the pH value of the sample, are marked. Since zymonic acid slowly decomposed into parapyruvate within a period of 24 hours_ENREF_19 (Montgomery et al., 1956), its peaks can be assigned from subtraction of one spectrum from the other in combination with standard NMR prediction software (ChemDraw®). The proton spectra show a buffered solution of zymonic acid in water and D₂O, once measured right after preparation of the solution (top right of Figure 5) and 24h later (bottom right of Figure 5).

Mass spectrometry confirmed the chemical formula of zymonic acid (see Figure 6). Thereby, Figure 6A shows the HR-MS-spectrum of the synthesized substance which confirms the total mass of the compound. Figure 6B shows the MS/MS-spectrum of the synthesized substance recorded after CID-fragmentation, wherein the assignment of the observed fragments confirms the assumed structure. Within the accuracy of the ion trap (±0.3 m/z), all peaks can be explained by elimination of carbon monoxide and carbon dioxide.

### Detection of tumor cell death due to pH change

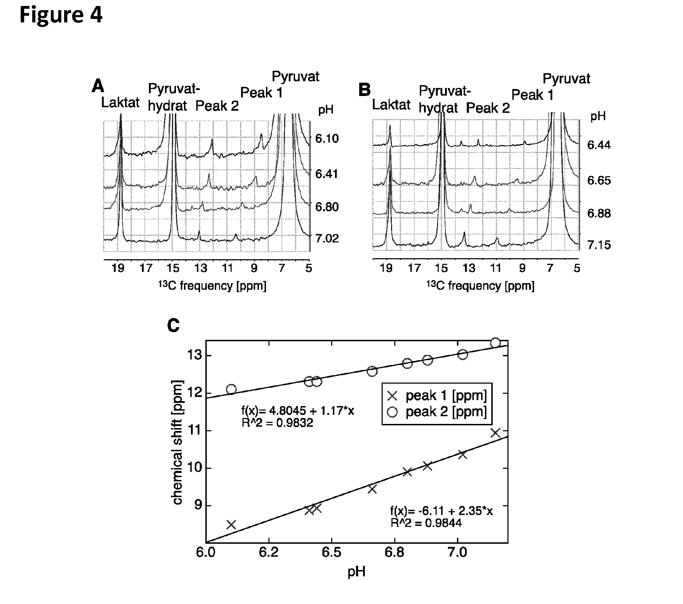

The pH dependent peaks 1 and 2 of zymonic acid were also observed in ¹³C-NMR spectra of hyperpolarized, ¹³C-labeled [1-¹³C]-pyruvate in MCF-7 tumor cells (see Figure 4).

Zymonic acid is formed from parapyruvate by a ring closure. In this process, the originally ¹³C-labeled carboxyl groups create a ¹³C-labeling of zymonic acid in positions 1 and 2 (see Figure 1). The pH dependent chemical shift of peak 1 of zymonic acid was determined to be approx. 2.35 ppm/pH in this measurement and approx. 1.17 ppm/pH for peak 2, which is in good agreement with the results from the thermally polarized and unlabeled zymonic acid (cf. Figure 2, i.e. 2.11 ppm/H and 1.11 ppm/pH, respectively).

The tumor cells were treated with Triton X-100 so that they gradually become necrotic with time and that pH decreases successively, similar to the case of a necrotic tumor. As an exemplary application, this pH change in tumor cells can be detected using our pH biosensor. The pH of the tumor cell suspension was determined immediately after the NMR-measurement using a pH electrode as a reference (see Figure 4 A,B). As is demonstrated in Figure 4C, two peaks, which can be assigned to the hyperpolarized zymonic acid, show a strong pH dependent linear change in chemical shift with up to 2.35 ppm/pH.

The features disclosed in the foregoing description, in the claims and/or in the accompanying drawings may, both separately and in any combination thereof, be material for realizing the invention in diverse forms thereof.

### REFERENCES

Aime S, Delli Castelli D, Terreno E. Novel pH-reporter MRI contrast agents. Angew Chem Int Ed Engl 2002;41:4334-6.
Ardenkjaer-Larsen JH, Fridlund B, Gram A, et al. Increase in signal-to-noise ratio of > 10,000 times in liquid-state NMR. Proceedings of the National Academy of Sciences of the United States of America 2003;100:10158-63.
Arnold DL, Matthews PM, Radda GK. Metabolic recovery after exercise and the assessment of mitochondrial function in vivo in human skeletal muscle by means of 31P NMR. Magnetic resonance in medicine : official journal of the Society of Magnetic Resonance in Medicine / Society of Magnetic Resonance in Medicine 1984;1:307-15.
Bloomer JL, Gross MA. Biosynthesis of Zymonic Acid in Trichosporon-Capitatum. J Chem Soc Chem Comm 1970:73-74.
Bloomer JL, Gross MA, Kappler FE, Pandey GN. Identity of Zymonic Acid with a Pyruvate Derivative. J Chem Soc Chem Comm 1970:1030.
Castelli DC, Ferrauto G, Cutrin JC, Terreno E, Aime S. In vivo maps of extracellular pH in murine melanoma by CEST-MRI. Magnetic resonance in medicine : official journal of the Society of Magnetic Resonance in Medicine / Society of Magnetic Resonance in Medicine 2013: 1-8.
Day SE, Kettunen MI, Gallagher FA, Hu DE, Lerche M, Wolber J, Golman K, Ardenkjaer-Larsen JH, Brindle KM. Detecting tumor response to treatment using hyperpolarized 13C magnetic resonance imaging and spectroscopy. Nat Med. 2007 Nov;13(11):1382-7.
De Graaf, Robin. In Vivo NMR Spectroscopy: Principles and Techniques, John Wiley & Sons; 2007.
De Jong MAKW. L'action de l'acid chlorhydrique sur l'acide pyruvique. Recueil des travaux chimiques des Pays-Bas 1901;20:81-101.
De Leon-Rodriguez LM, Lubag AJ, Malloy CR, Martinez GV, Gillies RJ, Sherry AD. Responsive MRI agents for sensing metabolism in vivo. Accounts of chemical research 2009;42:948-57.
Ernst, Richard. Principles of Nuclear Magnetic Resonance in One and Two Dimension (International Series of Monographs on Chemistry), Oxford University Press; 1997.
Gallagher FA, Kettunen MI, Day SE, Hu DE, Ardenkjaer-Larsen JH, Zandt Ri, Jensen PR, Karlsson M, Golman K, Lerche MH, Brindle KM. Magnetic resonance imaging of pH in vivo using hyperpolarized 13C-labelled bicarbonate. Nature. 2008 Jun 12;453(7197):940-3.
Gillies RJ, Raghunand N, Garcia-Martin ML, Gatenby RA. pH imaging. A review of pH measurement methods and applications in cancers. IEEE engineering in medicine and biology magazine : the quarterly magazine of the Engineering in Medicine & Biology Society 2004;23:57-64.
Hassan M, Riley J, Chernomordk V, et al. Fluorescence lifetime Imaging system for in vivo studies. Mol Imaging 2007;6:229-36.
Montgomery CM, Webb JL. Detection of a New Inhibitor of the Tricarboxylic Acid Cycle. Science 1954;120:843-4.
Montgomery CM, Webb JL. Metabolic studies on heart mitochondria. II. The inhibitory action of parapyruvate on the tricarboxylic acid cycle. The Journal of biological chemistry 1956;221:359-68.
Morikawa S, Inubushi T, Kito K, Kido C. pH mapping in living tissues: an application of in vivo 31P NMR chemical shift imaging. Magnetic resonance in medicine : official journal of the Society of Magnetic Resonance in Medicine / Society of Magnetic Resonance in Medicine 1993;29:249-51.
Nelson SJ, Ozhinsky E, Li Y, Park I, Crane J. Strategies for rapid in vivo 1H and hyperpolarized 13C MR spectroscopic imaging. J Magn Reson 2013;229:187-97.
Nelson SJ, Kurhanewicz J, Vigneron DB, Larson PE, Harzstark AL, Ferrone M, van Criekinge M, Chang JW, Bok R, Park I, Reed G, Carvajal L, Small EJ, Munster P, Weinberg VK, Ardenkjaer-Larsen JH, Chen AP, Hurd RE, Odegardstuen LI, Robb FJ, Tropp J, Murray JA. Metabolic Imaging of Patients with Prostate Cancer Using Hyperpolarized [1-13C]Pyruvate. Sci Transl Med. 2013 Aug 14;5(198):198ra108.
V. Rieke, K. Vigen, G. Sommer, B. Daniel, J. Pauly, K. Butts, Referenceless PRF Shift thermometry, MRM, 2004 Jun;51(6):1223-31.
Schroeder MA, Swietach P, Atherton HJ, et al. Measuring intracellular pH in the heart using hyperpolarized carbon dioxide and bicarbonate: a 13C and 31P magnetic resonance spectroscopy study. Cardiovascular research 2010;86:82-91.
Stodola FH, Shotwell OL, Lockwood LB. Zymonic Acid, a New Metabolic Product of Some Yeasts Grown in Aerated Culture .1. Structure Studies. J Am Chem Soc 1952;74:5415-8.
Vavere AL, Biddlecombe GB, Spees WM, et al. A Novel Technology for the Imaging of Acidic Prostate Tumors by Positron Emission Tomography. Cancer Res 2009;69:4510-6.
Zhang X, Lin Y, Gillies RJ. Tumor pH and its measurement. Journal of nuclear medicine : official publication, Society of Nuclear Medicine 2010;51:1167-70.

## Claims

1. Use of a compound with at least one pH-sensitive chemical shift for determining pH and/or measuring pH changes,
wherein the compound is selected from pyruvic acid and its metabolites, compounds produced from pyruvic acid after interaction with acid, and compounds comprising at least one enolic group whose pKₐ value is lowered through effects of at least one neighboring group into a physiological and/or pathological pH-range.

2. The use of claim 1, wherein the compound is ¹³C-labeled and preferably exhibits at least one pH-sensitive ¹³C chemical shift,
and/or wherein the at least one pH-sensitive chemical shift is pH-sensitive in a physiological and/or pathological pH range, preferably from about pH 5 to about pH 9,
and/or wherein the compound furthermore exhibits at least one chemical shift that is not pH-sensitive, preferably at least one pH-insensitive ¹³C chemical shift.

3. The use of claim 1 or 2, wherein the compound is selected from zymonic acid,
diethyl oxaloacetic acid,
pyruvic acid and its metabolites,
analogs of zymonic acid,
such as esters, ethers, amides, fluorinated analogs, deuterated analogs,
a compound that is produced from pyruvic acid after interaction with acid,
and their hydrates, salts, solutions, stereoisomers.

4. The use of any one of claims 1 to 3, wherein the compound is hyperpolarized, preferably by dynamic nuclear polarization (DNP),
and/or wherein the compound has a pKₐ value in a physiological and/or pH range, preferably from about 5 to about 9, and/or the carbon(s) belonging to the pH-sensitive chemical shift(s) of the compound exhibit(s) a long longitudinal relaxation time T₁.

5. A biosensor for determining pH and/or measuring pH changes,
comprising
at least one compound with at least one pH-sensitive chemical shift as defined in any one of claims 1 to 4,
optionally, a reference compound,
optionally, one or several pharmaceutically acceptable carriers and/or excipients,
wherein, preferably, the reference compound is a compound which does not exhibit pH-sensitive chemical shift(s) in an NMR spectrum, preferably the reference compound is ¹³C-labeled and preferably exhibits at least one pH-insensitive ¹³C chemical shift.

6. The compound of any of claims 1 to 4 or the biosensor of claim 5 for use in *in-vivo* magnetic resonance imaging (MRI), magnetic resonance spectroscopy (MRS) or magnetic resonance tomography (MRT).

7. The compound of any of claims 1 to 4 or the biosensor of claim 5 for use in diagnosing and/or monitoring treatment of a disease causing changes in pH,
wherein a disease causing changes in pH is preferably selected from cancers, inflammation, ischemia, renal failure and chronic obstructive pulmonary disease

8. The compound or biosensor of claim 7, wherein the imaging is real-time,
preferably comprising the resolution of the spatial pH distribution,
more preferably comprising the use of frequency encoding techniques, such as chemical shift imaging (CSI) and phase sensitive encodings of chemical shifts.

9. Use of the compound of any of claims 1 to 4 as pH sensor for *in-vitro* NMR-spectroscopy,
preferably comprising response-to-treatment monitoring of treatments applied to cell lines.

10. An *in-vitro* method for determining pH and/or measuring pH changes,
comprising the steps of
(i) providing a sample,
(ii) adding a compound with at least one pH-sensitive chemical shift of any of claims 1 to 4 or a biosensor of claim 5 to the sample,
(iii) performing magnetic resonance imaging (MRI) or magnetic resonance spectroscopy (MRS) and thereby determining the pH or pH changes of or in the sample by obtaining a chemical shift difference between at least one pH-sensitive chemical shift of the compound and a pH-independent chemical shift, such pH-independent chemical shift acting as a reference chemical shift, or by measurement of the absolute chemical shift, or by measuring chemical shift differences involving at least one pH-sensitive shift.
wherein the sample is preferably a cell culture sample (such as derived from a human or non-human body, *ex vivo* tissue, cell culture),
and/or wherein step (iii) is preferably carried out in an MRI scanner machine with MRS or MRSI capabilities or in a NMR spectrometer.

11. An *in vivo* method for determining pH and/or measuring pH changes, preferably in real-time,
comprising the steps of
(i) applying or administering a compound with at least one pH-sensitive chemical shift of any of claims 1 to 8, a biosensor of any of claims 5-8 to the body of a patient or non-human animal,
(ii) performing magnetic resonance imaging (MRI) and thereby determining one or several pH values or pH changes of or in the body of said patient or non-human animal by obtaining a chemical shift difference between at least one pH-sensitive chemical shift of the compound and a pH-independent chemical shift, such pH-independent chemical shift acting as a reference chemical shift, or by measurement of the absolute chemical shift, or by measuring chemical shift differences involving at least one pH-sensitive chemical shift.
wherein the patient can preferably be diagnosed with a disease causing changes in pH or the treatment of a disease causing changes in pH can be monitored,
wherein a disease causing changes in pH is preferably selected from cancers, inflammation, ischemia, renal failure and chronic obstructive pulmonary disease.

12. The method of claim 10 or 11, wherein the pH-independent chemical shift (reference chemical shift) is from the same compound, i.e. the compound with at least one pH-sensitive chemical shift, or from another substance, and is used as a pH-independent reference.

13. A method of diagnosing and/or monitoring treatment of a disease causing changes in pH,
comprising the steps of
(i) applying or administering a compound with at least one pH-sensitive chemical shift of any of claims 1-8 or a biosensor of any of claims 5-8 to the body of a patient or non-human animal,
(ii) performing magnetic resonance imaging (MRI) or magnetic resonance spectroscopy (MRS) and thereby determining and or several pH values or pH changes of or in the body of said patient or non-human animal by obtaining the chemical shift difference between at least one pH sensitive chemical shift of the compound and a pH-independent chemical, such pH-independent chemical shift acting as a reference chemical shift, or by measurement of the absolute chemical shift, or by measuring chemical shift difference involving at least one pH-sensitive chemical shift over time,
(iii) calculating pH maps based on spatially resolved pH values or pH changes determined in the step (ii).
wherein a disease causing changes in pH is preferably selected from cancers, inflammation, ischemia, renal failure and chronic obstructive pulmonary disease,
wherein step (iii) preferably comprises
comparing said relative chemical shifts to predetermined calibration curves of the compound with at least one pH-sensitive chemical shift in solutions with known pH,
and/or said method furthermore comprises
hyperpolarizing the compound with at least one pH-sensitive chemical shift before application or administration to the body of the patient.

14. The method of claim 13, comprising magnetic resonance spectroscopy (MRS) or magnetic resonance tomography (MRT),
and/or wherein the imaging is real-time.

15. The method of any one of claims 10 to 14, comprising the resolution of a spatial pH distribution,
preferably comprising the use of frequency encoding techniques, such as comprising chemical shift imaging (CSI) and phase sensitive encodings of chemical shifts.

16. Use of the compound of any of claims 1 to 4 or the biosensor of claim 5 in quality control of food or in the examination of plants and organisms.
